# EUROPEAN PATENT SPECIFICATION

(11) **EP 2 593 190 B1**
(45) Date of publication and mention of the grant of the patent: **23.08.2017**
(21) Application number: 11738540.1
(22) Date of filing: 12.07.2011
(51) Int. Cl.: A61Q 9/02, A61K 8/97, A61K 8/42, A61K 8/63

(54) **PERSONAL CARE COMPOSITIONS COMPRISING A MULTI-ACTIVE SYSTEM FOR DOWN REGULATING CYTOKINES IRRITATION**
KÖRPERPFLEGEZUSAMMENSETZUNG MIT EINEM MEHRFACH AKTIVEN SYSTEM ZUR HERUNTERREGULIERUNG DER REIZUNG DURCH ZYTOKINE
COMPOSITIONS DE SOIN PERSONNEL COMPRENANT UN SYSTÈME MULTIACTIF POUR LA RÉGULATION NÉGATIVE DE L'IRRITATION DUE AUX CYTOKINES

(30) Priority: 16.07.2010 US 364932 P
(43) Date of publication of application: 22.05.2013
(73) Proprietor: The Gillette Company LLC, Boston, MA 02127 (US)
(72) Inventor: COFFINDAFFER, Timothy, Woodrow, Maineville Ohio 45039 (US); HEATH, Benjamin, Parker, Cincinnati Ohio 45231 (US); KEMP, Helen, Rochelle, Glendale Ohio 45246 (US); WILLICUT, Robert, John, Liberty Township Ohio 45011 (US)
(74) Representative: Kohol, Sonia
(86) International application number: PCT/US2011/043613
(87) International publication number: WO 2012/009298

(56) References cited:
- WO-A1-2009/115455
- WO-A2-97/31620
- US-A- 5 080 901
- US-A1- 2004 185 022
- US-A1- 2007 154 425
- US-A1- 2007 224 154
- US-A1- 2008 206 359
- DATABASE GNPD [Online] MINTEL; September 2009 (2009-09), "Renewal & Vitality Daily Face Moisturizer", XP002723066, Database accession no. 1172095
- DATABASE GNPD [Online] MINTEL; April 2009 (2009-04), "Ultimate moisture cream", XP002723067, Database accession no. 1080356
- DATABASE GNPD MINTEL; May 2006 (2006-05), "Facial Cleansing Gel", XP002723068, Database accession no. 533263
- DATABASE GNPD [Online] MINTEL; July 2005 (2005-07), "Hydrating Toner", XP002723069, Database accession no. 10226628

## Description

### BACKGROUND OF THE INVENTION

Manufacturers of skin care compositions have made many attempts to incorporate various ingredients into their products to provide benefits such as anti-aging; skin health; anti-inflammation. The ingredients mentioned for these types of purposes are many and the disclosures of such use are prolific. *See e.g.* U.S. Patent Publ. Nos. 2010/0055138; 2009/123576; 2008/0069784; 2008/0199533; 2008/0003188; 2007/0274932; 2006/0257386; 2006/0165643; 2005/0019356; 2002/0022040; and 2002/0197228. Another recent disclosure purports to measure methods of reducing skin irritation via IL1-α reduction testing. *See* U.S. Patent Publ. No. 2007/0224154 in Table 1. One known class of compositions which have been described to provide desirable anti-inflammation benefits is a corticosteroid component such as a clobetasol derivative. *See* U.S. Patent No. 4,343,798. Clobetasol derivatives, however, may not be desirable for all types of product executions. Despite these many different types of skin care compositions, there remains a desire to find a composition suitable for use on the skin which delivers skin irritation reduction performance similar to clobetasol derivatives.

### SUMMARY OF THE INVENTION

The present invention provides for a method of reducing or controlling skin irritation by applying a personal care composition of the present invention onto skin to form a treated surface, comprising from about 0.001% to about 8% by weight of a multi-active system for down regulating cytokines, said multi active system comprising: an extract of *camellia sinensis* (such as white tea extract); panthenol; and a glycyrrhizinate salt; and from about 50% to about 99.99% by weight of a carrier. The treated surface can then be subjected to hair removal (i.e. shaving or other) or the treatment can follow the hair removal process.

### BRIEF DESCRIPTION OF THE FIGURES

FIG. 1 is a chart showing IL1-α reduction values for given treatments.
FIG. 2 is a table of various examples in accordance with the present invention.
FIG. 3 is another table of various examples in accordance with the present invention.

### DETAILED DESCRIPTION OF THE INVENTION

The personal care composition applied in the present invention comprises a multi-active system for down regulating cytokines. Without intending to be bound by theory, it is believed that by including multiple actives the ability of each active to reduce skin inflammation is increased such that the combined use of the multiple actives exceeds the benefit obtained by using each active separately. The multi-active system for down regulating cytokines comprises at least three actives: an extract of *camellia sinesis,* panthenol, and glycyrrhizinate salt. The personal care composition applied in the present invention comprises from about 0.001% to about 8%, or from about 0.01% to about 5%, or from about 0.1% to about 3%, or from about 0.2% to about 1.5%, or from about 0.25% to about 1.0 % by weight of the multi-active system, by weight.

### 1. Multi-Active System for Down Regulating Cytokines

The multi-active system for down regulating cytokines comprises at least an extract of *camellia sinesis;* panthenol; and glycyrrhizinate salt. Each of these actives (and any other ingredients in the present invention, are included in a safe and effective amount for topical application. The level of the extract of *camellia sinesis* is from 5% to 50%, alternatively from 10% to 25% by weight of said multi-active system. The level of glycyrrhizinate salt is from 5% to 50%, alternatively from 20% to 40% by weight of said multi-active system. The level of panthenol is from 15% to 80%, alternatively from 40% to 70% by weight of said multi-active system.

In one embodiment, the level of panthenol is higher than the level of the extract of *camellia sinesis,* or the level of glycyrrhizinate salt, alone or in combination. In another embodiment, the level of panthenol is higher than the level of the extract of *camellia sinesis.* In one embodiment, said multi-active system comprises a ratio of said extract of *camellia sinesis* to glycyrrhizinate salt to panthenol is from about 10:5:1 to about 1:5:10 by weight. In one embodiment, the ratio is from about 7:3:1 to about 1:3:7 by weight. In yet another embodiment, the actives in the multi-active system are provided in close proportion, for example each within about 10% of the others, or within about 5 %, or within about 1%, by weight of the multi-active system. In another embodiment, the multi-active system consists of these three actives.

Without intending to be bound by theory, it is believed that such a multi-active system provides suitable skin irritation reduction as can be demonstrated via interleukin 1-α ("IL1-α") reduction testing as shown in Table

### a. Extract of Camelia Sinesis

In some embodiments, extracts of *camellia sinensis* that can be used include but are not limited to green tea extract, black tea extract, white tea extract, individual components of green tea extract, black tea, and/or white tea extract (including but not limited to epicatechin, catechin, epigallocatechin gallate or EGCG, and the like), and any combination of the above. In one preferred embodiment, the extract of *camellia sinensis* is an extract of white tea.

White tea extract usually is obtained from a tea that is fermented only to about 2%. This fermentation occurs during the wilting process in a natural manner. In the case of white tea, the delicate flower buds of this species are dried. During drying they become whitish, hence the name of this tea. It is also referred to as a special type of green tea.

Essential constituents of white tea extract are xanthines and catechols, especially gallocatechol, epigallocatechol, epicatechol, epigallocatechol gallate, galllocatechol gallate, and epicatechol gallate.

Commercially available sources of white tea extract include WHITE TEA COMPLEX by Barnet Products Corporation, WHITE TEA EXTRACT by Carrubba, and EXTRAPONE® WHITE TEA by Cognis.

### b. Panthenol

The multi-active system comprises panthenol. Panthenol is the alcohol analog of pantothenic acid (vitamin B₅), and is thus a provitamin of B₅. In organisms it is quickly oxidized to pantothenate. Panthenol is a highly viscous transparent liquid at room temperature, but salts of pantothenic acid (for example sodium pantothenate) are powders (typically white). It is soluble in water, alcohol and propylene glycol, soluble in ether and chloroform, and slightly soluble in glycerin.

Panthenol comes in two enantiomers, D and L. Only D-panthenol (*dexpanthenol*) is biologically active, however both forms have moisturizing properties. For cosmetic use, panthenol comes either in D form, or as a racemic mixture of D and L (DL-panthenol). Panthenol's expanded chemical formula is: HO-CH₂-C(CH₃)₂-CH(OH)-CONH-CH₂CH₂CH₂-OH. It has been reported in Ebner F, Heller A, Rippke F, Tausch I. "Topical use of dexpanthenol in skin disorders". American journal of clinical dermatology. 2002;3(6):427-33 that Panthenol in lotions, can improve hydration, reduce itching and inflammation of the skin and accelerates and improve healing of epidermal wounds.

Other names for panthenol include:
- Butanamide, 2,4-dihydroxy-*N*-(3-hydroxypropyl)-3,3-dimethyl-, (R)-
- Butyramide, 2,4-dihydroxy-*N*-(3-hydroxypropyl)-3,3-dimethyl-, D-(+)-
- Butanamide, 2,4-dihydroxy-*N*-(3-hydroxypropyl)-3,3-dimethyl-, (2*R*)-
- D-Panthenol
- Dexpanthenol (DCIR)
- Dexpanthenolum
- Propanolamine, *N*-pantoyl-
- d-Pantothenyl alcohol
- Bepanthen

### c. Glycyrrhizinate salt

Glycyrrhizinate salt is widely used anti-inflammatory agent isolated from the licorice root. It is metabolized to glycyrrhetic acid, which inhibits 11 beta-hydroxysteroid dehydrogenase and other enzymes involved in the metabolism of corticosteroids. In one embodiment, the glycyrrhizinate salt is a dipotassium salt, as shown below: 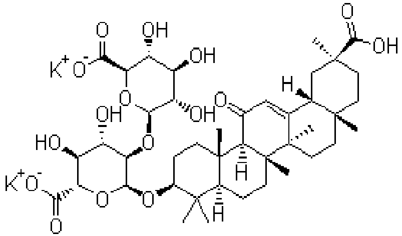 It may be purchased from: Mafco, Sandream or Barnet. Other salts of glycyrrhizinate may also be used, including but not limited to ammonium, sodium, and alkanolamine (e.g., triethanolamine) salts.

### 2. Optional Skin Care Actives

In one embodiment, the personal care composition further comprises one or more additional skin care actives which are commonly used in cosmetic and personal care compositions on the market today. Each of the one or more optional skin care actives can be provided at from about 0.001% to about 10%, or from about 0.1% to about 5% by weight of the composition. Non-limiting examples of suitable actives include one or more of: Bisabolol and Ginger root; sodium polyethylene glycol 7 olive oil carboxylate; Lauryl p-Cresol Ketoxime, 4-(1-Phenylethyl) 1,3-benzenediol, Lupin (Lupinus albus) oil & wheat (Triticum vulgare) germ oil unsaponifiables, Hydrolyzed lupin protein, Extract of L-lysine and L-arginine peptides, Oil soluble vitamin C, Evodia rutaecarpa fruit extract, Zinc pidolate and zinc PCA, Alpha-linoleic acid, p-thymol, and combinations thereof; at least one additional skin and/or hair care active selected from the group consisting of sugar amines, vitamin B₃, retinoids, hydroquinone, peptides, farnesol, phytosterol, dialkanoyl hydroxyproline, hexamidine, salicylic acid, N-acyl amino acid compounds, sunscreen actives, water soluble vitamins, oil soluble vitamins, hesperedin, mustard seed extract, glycyrrhizic acid, glycyrrhetinic acid, carnosine, Butylated Hydroxytoluene (BHT) and Butylated Hydroxyanisole (BHA), menthyl anthranilate, cetyl pyridinium chloride, tetrahydrocurmin, vanillin or its derivatives, ergothioneine, melanostatine, sterol esters, idebenone, dehydroacetic acid, Licohalcone A, creatine, creatinine, feverfew extract, yeast extract (e.g., Pitera®), beta glucans, alpha glucans, diethylhexyl syringylidene malonate, erythritol, p-cymen-7-ol, benzyl phenylacetate, 4-(4-methoxyphenyl)butan-2-one, ethoxyquin, tannic acid, gallic acid, octadecenedioic acid, p-cymen-5-ol, methyl sulfonyl methane, an avenathramide compound, fatty acids (especially poly-unsaturated fatty acids), anti-fungal agents, thiol compounds (e.g., N-acetyl cysteine, glutathione, thioglycolate), other vitamins (vitamin B 12), beta-carotene, ubiquinone, amino acids, their salts, their derivatives, their precursors, and/or combinations thereof; and a dermatologically acceptable carrier. These and other potentially suitable actives are described in greater detail in U.S. Patent Publication No. 2008/0069784.

In another embodiment, the personal care composition further comprising from about 0.001% to about 1% of methyl naphthalenyl ketone. The methyl naphthalenyl ketone can be a 1-(1,2,3,4,5,6,7,8-octahydro-2,3,8,8-tetramethyl-2naphthalenyl)-ethan-1-one molecule or an isomer or derivative thereof. Commercially available as Iso-E-Super from IFF of New York.

In yet another embodiment, the personal care composition further comprising from about 0.001% to about 1%, preferably from about 0.05% to about 0.5% of a cooling agent. Preferred cooling agents but not limited to are menthol, CoolAct 10, menthyl lactate, and combinations thereof.

### 3. Carrier

The personal care compositions applied in the present invention also comprise a carrier for the multi-active system for down regulating cytokines. The carrier is preferably dermatologically acceptable, meaning that the carrier is suitable for topical application to the keratinous tissue, has good aesthetic properties, is compatible with the actives of the present invention and any other components, and will not cause any safety or toxicity concerns. In one embodiment, the personal care composition comprises from about 50% to about 99.99%, preferably from about 60% to about 99.9%, more preferably from about 70% to about 98%, and even more preferably from about 80% to about 95% of the carrier by weight of the composition.

The carrier can be in a wide variety of forms. For example, emulsion carriers, including, but not limited to, oil-in-water, water-in-oil, water-in-oil-in-water, and oil-in-water-in-silicone emulsions, are useful herein.

Preferred carriers comprise an emulsion such as oil-in-water emulsions and water-in-oil emulsions, e.g., silicone-in-water or water-in-silicone emulsions. As will be understood by the skilled artisan, a given component will distribute primarily into either the water or oil phase, depending on the water solubility/dispensability of the component in the composition. Oil-in-water emulsions are especially preferred.

Emulsions applied according to the present invention generally contain a solution as described above and a lipid or oil. Lipids and oils may be derived from animals, plants, or petroleum and may be natural or synthetic. Preferred emulsions also contain a humectant, such as glycerin. Emulsions will preferably further contain from about 0.1% to about 10%, more preferably from about 0.2% to about 5%, of an emulsifier, based on the weight of the composition. Emulsifiers may be nonionic, anionic or cationic. The emulsifier can be a polymer, a surfactant or a mixture thereof. Suitable emulsifiers are disclosed in, for example, U.S. Patent 3,755,560, 4,421,769, and McCutcheon's Detergents and Emulsifiers, North American Edition, pages 317-324 (1986).

### a. Water-in-Oil emulsion

Water in oil emulsions are characterized as having a continuous hydrophobic, water insoluble oil phase and a water phase dispersed therein. The "oil phase" can contain oil, silicone or mixtures thereof. The distinction of whether the emulsion is characterized as a water-in-oil or water-in-silicone emulsion is a function of whether the oil phase is composed of primarily oil or silicone. A preferred example of a water-in-silicone emulsion is described below.

### 1. Continuous silicone phase

Preferred water-in-silicone emulsions applied in the present invention comprise from about 1% to about 60%, preferably from about 5% to about 40%, more preferably from about 10% to about 30%, by weight of a continuous silicone phase. The continuous silicone phase exists as an external phase that contains or surrounds the discontinuous aqueous phase described hereinafter.

The continuous silicone phase contains a silicone elastomer and/or polyorganosiloxane oil. The continuous silicone phase of these preferred emulsions comprises between about 50% and about 99.9% by weight of organopolysiloxane oil and less than about 50% by weight of a non-silicone oil. In a preferred embodiment, the continuous silicone phase comprises at least about 50%, preferably from about 60% to about 99.9%, more preferably from about 70% to about 99.9%, and even more preferably from about 80% to about 99.9%, polyorganosiloxane oil by weight of the continuous silicone phase, and up to about 50% non-silicone oils, preferably less about 40%, more preferably less than about 30%, even more preferably less than about 10%, and still more preferably less than about 2%, by weight of the continuous silicone phase.

### 2. Polyorganopolysiloxane Oil

The organopolysiloxane oil for use in the composition may be volatile, non-volatile, or a mixture of volatile and non-volatile silicones. The term "nonvolatile" as used in this context refers to those silicones that are liquid under ambient conditions and have a flash point (under one atmospheric of pressure) of or greater than about 100°C. The term "volatile" as used in this context refers to all other silicone oils. Suitable organopolysiloxanes can be selected from a wide variety of silicones spanning a broad range of volatilities and viscosities. Examples of suitable organopolysiloxane oils include polyalkylsiloxanes, cyclic polyalkylsiloxanes, and polyalkylarylsiloxanes.

Suitable polyalkylsiloxanes include polyalkylsiloxanes with viscosities of from about 0.5 to about 1,000,000 centistokes at 25°C. Commercially available polyalkylsiloxanes include polydimethylsiloxanes, which are also known as dimethicones, examples of which include the Vicasil^{®} series sold by General Electric Company and the Dow Corning^{®} 200 series sold by Dow Corning Corporation. Cyclic polyalkylsiloxanes suitable for use in the composition include those commercially available such as Dow Corning^{®} 244, Dow Corning^{®} 344 fluid, and Dow Corning^{®} 345 fluid.

Also useful are materials such as trimethylsiloxysilicate, which is a polymeric material corresponding to the general chemical formula [(CH₂)₃SiO_{1/2}]ₓ[SiO₂]_{y}, wherein x is an integer of from about 1 to about 500 and y is an integer of from about 1 to about 500. A commercially available trimethylsiloxysilicate is sold as a mixture with dimethicone as DC^{®} 593 fluid.

Dimethiconols are also suitable for use in the composition. These compounds can be represented by the chemical formulas R₃SiO[R₂SiO]ₓSiR₂OH and HOR₂SiO[R₂SiO]ₓSiR₂OH wherein R is an alkyl group (preferably R is methyl or ethyl) and x is an integer of from 0 to about 500, chosen to achieve the desired molecular weight. Commercially available dimethiconols are typically sold as mixtures with dimethicone or cyclomethicone (e.g. Dow Corning^{®} 1401, 1402, and 1403 fluids).

Polyalkylaryl siloxanes are also suitable for use in the composition, particularly those having viscosities of from about 15 to about 65 centistokes at 25°C.

Preferred for use herein are organopolysiloxanes selected from the group consisting of polyalkylsiloxanes, alkyl substituted dimethicones, cyclomethicones, trimethylsiloxysilicates, dimethiconols, polyalkylaryl siloxanes, and mixtures thereof. More preferred for use herein are polyalkylsiloxanes and cyclomethicones. Preferred polyalkylsiloxanes are dimethicones.

As stated above, the continuous silicone phase may contain one or more non-silicone oils. Suitable non-silicone oils have a melting point of about 25°C or less under about one atmosphere of pressure. Examples of non-silicone oils suitable for use in the continuous silicone phase are known in the chemical arts in topical personal care products which can be in the form of emulsions, e.g., mineral oil, vegetable oils, synthetic oils, semisynthetic oils, fatty acid esters, etc.

### 3. Silicone Elastomer

The compositions applied in the present invention may also include from about 0.1% to about 30%, by weight of the composition, of a silicone elastomer component. Preferably, the composition includes from about 1% to about 30%, more preferably from about 2% to about 20%, by weight of the composition, of the silicone elastomer component.

Suitable for use herein are silicone elastomers, which can be emulsifying or non-emulsifying crosslinked siloxane elastomers or mixtures thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester.

Addition reaction-curing organopolysiloxane compositions are preferred for their rapid curing rates and excellent uniformity of curing. A particularly preferred addition reaction-curing organopolysiloxane composition is prepared from:
(A) an organopolysiloxane having at least 2 lower alkenyl groups in each molecule;
(B) an organopolysiloxane having at least 2 silicon-bonded hydrogen atoms in each molecule; and
(C) a platinum-type catalyst.

In one embodiment the composition includes an emulsifying crosslinked organopolysiloxane elastomer, a non-emulsifying crosslinked organopolysiloxane elastomer, or a mixture thereof. The term "non-emulsifying," as used herein, defines crosslinked organopolysiloxane elastomers from which polyoxyalkylene units are absent. The term "emulsifying," as used herein, means crosslinked organopolysiloxane elastomers having at least one polyoxyalkylene (e.g., polyoxyethylene or polyoxypropylene) unit. Preferred emulsifying elastomers herein include polyoxyalkylene modified elastomers formed from divinyl compounds, particularly siloxane polymers with at least two free vinyl groups, reacting with Si-H linkages on a polysiloxane backbone. Preferably, the elastomers are dimethyl polysiloxanes crosslinked by Si-H sites on a molecularly spherical MQ resin. Emulsifying crosslinked organopolysiloxane elastomers can notably be chosen from the crosslinked polymers described in US Patents 5,412,004, 5,837,793, and 5,811,487. An emulsifying elastomer comprising dimethicone copolyol crosspolymer (and) dimethicone is available from Shin Etsu as KSG-21.

Advantageously, the non-emulsifying elastomers are dimethicone/vinyl dimethicone crosspolymers. Such dimethicone/vinyl dimethicone crosspolymers are supplied by a variety of suppliers including Dow Corning (DC 9040 and DC 9041), General Electric (SFE 839), Shin Etsu (KSG-15, 16, 18 [dimethicone/phenyl vinyl dimethicone crosspolymer]), and Grant Industries (GRANSIL™ line of elastomers). Cross-linked organopolysiloxane elastomers useful in the present invention and processes for making them are further described in U.S. Patent 4,970,252, U.S. Patent 5,760,116, and U.S. Patent 5,654,362. Additional crosslinked organopolysiloxane elastomers useful in the present invention are disclosed in Japanese Patent Application JP 61-18708, assigned to Pola Kasei Kogyo KK.

Commercially available elastomers preferred for use herein are Dow Corning's 9040 silicone elastomer blend, Shin Etsu's KSG-21, and mixtures thereof.

### 4. Carrier for Silicone Elastomer

The topical compositions applied in the present invention may include from about 1% to about 80%, by weight of the composition, of a suitable carrier for the for the crosslinked organopolysiloxane elastomer component described above. The carrier, when combined with the cross-linked organopolysiloxane elastomer particles of the present invention, serves to suspend and swell the elastomer particles to provide an elastic, gel-like network or matrix. The carrier for the cross-linked siloxane elastomer is liquid under ambient conditions, and preferably has a low viscosity to provide for improved spreading on skin.

Concentrations of the carrier in the cosmetic compositions applied in the present invention will vary primarily with the type and amount of carrier and the cross-linked siloxane elastomer employed. Preferred concentrations of the carrier are from about 5% to about 50%, more preferably from about 5% to about 40%, by weight of the composition.

The carrier for the cross-linked siloxane elastomer includes one or more liquid carriers suitable for topical application to human skin. These liquid carriers may be organic, silicone-containing or fluorine-containing, volatile or non-volatile, polar or non-polar, provided that the liquid carrier forms a solution or other homogenous liquid or liquid dispersion with the selected cross-linked siloxane elastomer at the selected siloxane elastomer concentration at a temperature of from about 28°C. to about 250°C., preferably from about 28°C. to about 100°C., preferably from about 28° C. to about 78° C. The term "volatile" as used herein refers to all materials that are not "non-volatile" as previously defined herein. The phrase "relatively polar" as used herein means more polar than another material in terms of solubility parameter; i.e., the higher the solubility parameter the more polar the liquid. The term "non-polar" typically means that the material has a solubility parameter below about 6.5 (cal/cm³)^{0.5}.

### 5. Non-polar, Volatile Oils

The non-polar, volatile oil tends to impart highly desirable aesthetic properties to the compositions of the present invention. Consequently, the non-polar, volatile oils are preferably utilized at a fairly high level. Non-polar, volatile oils particularly useful in the present invention are silicone oils; hydrocarbons; and mixtures thereof. Such non-polar, volatile oils are disclosed, for example, in Cosmetics, Science, and Technology, Vol. 1, 27-104 edited by Balsam and Sagarin, 1972. Examples of preferred non-polar, volatile hydrocarbons include polydecanes such as isododecane and isodecane (e.g., Permethyl-99A which is available from Presperse Inc.) and the C7 -C8 through C12 -C15 isoparaffins (such as the Isopar Series available from Exxon Chemicals). Particularly preferred volatile silicone oils are selected from cyclic volatile silicones with formula: 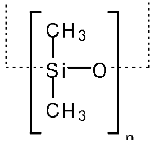 wherein n is from about 3 to about 7; and linear volatile silicones with formula:

(CH₃)₃ Si--O--[Si(CH₃)₂-O]ₘ --Si(CH₃)₃

wherein m is from about 1 to about 7. Linear volatile silicones generally have a viscosity of less than about 5 centistokes at 25° C., whereas the cyclic silicones have viscosities of less than about 10 centistokes at 25° C. Highly preferred examples of volatile silicone oils include cyclomethicones of varying viscosities, e.g., Dow Corning 200, Dow Corning 244, Dow Corning 245, Dow Corning 344, and Dow Corning 345, (from Dow Corning Corp.); SF-1204 and SF-1202 Silicone Fluids (from G.E. Silicones), GE 7207 and 7158 (from General Electric Co.); and SWS-03314 (from SWS Silicones Corp.).

### f. Relatively Polar, Non-volatile oils

The non-volatile oil is "relatively polar" as compared to the non-polar, volatile oil discussed above. Therefore, the non-volatile co-carrier is more polar (i.e., has a higher solubility parameter) than at least one of the non-polar, volatile oils. Relatively polar, non-volatile oils potentially useful in the present invention are disclosed, for example, in Cosmetics, Science, and Technology, Vol. 1, 27-104 edited by Balsam and Sagarin, 1972; U.S. Patents 4,202,879 and 4,816,261. Relatively polar, non-volatile oils useful in the present invention are preferably selected from silicone oils; hydrocarbon oils; fatty alcohols; fatty acids; esters of mono and dibasic carboxylic acids with mono and polyhydric alcohols; polyoxyethylenes; polyoxypropylenes; mixtures of polyoxyethylene and polyoxypropylene ethers of fatty alcohols; and mixtures thereof.

### 6. Non-polar, Non-volatile oils

In addition to the liquids discussed above, the carrier for the cross-linked siloxane elastomer may optionally include non-volatile, non-polar oils. Typical non-volatile, non-polar emollients are disclosed, for example, in Cosmetics, Science, and Technology, Vol. 1, 27-104 edited by Balsam and Sagarin, 1972; U.S. Patents 4,202,879 and 4,816,261. The non-volatile oils useful in the present invention are essentially non-volatile polysiloxanes, paraffinic hydrocarbon oils, and mixtures thereof.

### 7. Dispersed aqueous phase

The topical compositions applied in the present invention may comprise from about 30% to about 90%, more preferably from about 50% to about 85%, and even more preferably from about 70% to about 80% of a dispersed aqueous phase. In emulsion technology, the term "dispersed phase" is a term well-known to one skilled in the art which means that the phase exists as small particles or droplets that are suspended in and surrounded by a continuous phase. The dispersed phase is also known as the internal or discontinuous phase. The dispersed aqueous phase is a dispersion of small aqueous particles or droplets suspended in and surrounded by the continuous silicone phase described hereinbefore.

The aqueous phase can be water, or a combination of water and one or more water soluble or dispersible ingredients. Nonlimiting examples of such optional ingredients include thickeners, acids, bases, salts, chelants, gums, water-soluble or dispersible alcohols and polyols, buffers, preservatives, sunscreening agents, colorings, and the like.

The topical compositions applied in the present invention will typically comprise from about 25% to about 90%, preferably from about 40% to about 85%, more preferably from about 60% to about 80%, water in the dispersed aqueous phase by weight.

### 8. Emulsifier for dispersing the aqueous phase

The water-in-silicone emulsions applied in the present invention preferably comprise an emulsifier. In one embodiment, the composition contains from about 0.1% to about 10% emulsifier, more preferably from about 0.2% to about 7.5%, even more preferably from about 0.5% to about 5%, emulsifier by weight of the composition. The emulsifier helps disperse and suspend the aqueous phase within the continuous silicone phase.

A wide variety of emulsifying agents can be employed herein to form the preferred water-in-silicone emulsion. Known or conventional emulsifying agents can be used in the composition, provided that the selected emulsifying agent is chemically and physically compatible with essential components of the composition, and provides the desired dispersion characteristics. Suitable emulsifiers include silicone emulsifiers, non-silicon-containing emulsifiers, and mixtures thereof, known by those skilled in the art for use in topical personal care products. Preferably these emulsifiers have an HLB value of less than about 14, more preferably from about 2 to about 14, and even more preferably from about 4 to about 14. Emulsifiers having an HLB value outside of these ranges can be used in combination with other emulsifiers to achieve an effective weighted average HLB for the combination that falls within these ranges.

Silicone emulsifiers are preferred. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols.

Nonlimiting examples of dimethicone copolyols and other silicone surfactants useful as emulsifiers herein include polydimethylsiloxane polyether copolymers with pendant polyethylene oxide side chains, polydimethylsiloxane polyether copolymers with pendant polypropylene oxide side chains, polydimethylsiloxane polyether copolymers with pendant mixed polyethylene oxide and polypropylene oxide side chains, polydimethylsiloxane polyether copolymers with pendant mixed poly(ethylene)(propylene)oxide side chains, polydimethylsiloxane polyether copolymers with pendant organobetaine side chains, polydimethylsiloxane polyether copolymers with pendant carboxylate side chains, polydimethylsiloxane polyether copolymers with pendant quaternary ammonium side chains; and also further modifications of the preceding copolymers containing pendant C2-C30 straight, branched, or cyclic alkyl moieties. Examples of commercially available dimethicone copolyols useful herein sold by Dow Corning Corporation are Dow Corning^{®} 190, 193, Q2-5220, 2501 Wax, 2-5324 fluid, and 3225C (this latter material being sold as a mixture with cyclomethicone). Cetyl dimethicone copolyol is commercially available as a mixture with polyglyceryl-4 isostearate (and) hexyl laurate and is sold under the tradename ABIL^{®} WE-09 (available from Goldschmidt). Cetyl dimethicone copolyol is also commercially available as a mixture with hexyl laurate (and) polyglyceryl-3 oleate (and) cetyl dimethicone and is sold under the tradename ABIL^{®} WS-08 (also available from Goldschmidt). Other nonlimiting examples of dimethicone copolyols also include lauryl dimethicone copolyol, dimethicone copolyol acetate, diemethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate.

Among the non-silicone-containing emulsifiers useful herein are various non-ionic and anionic emulsifying agents such as sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, soaps, and mixtures thereof. Other suitable emulsifiers are described, for example, in McCutcheon's, Detergents and Emulsifiers, North American Edition (1986), published by Allured Publishing Corporation; U.S. Patent Nos. 5,011,681, 4,421,769, and 3,755,560.

### b. Oil-in-Water Emulsions

Other preferred topical carriers include oil-in-water emulsions, having a continuous aqueous phase and a hydrophobic, water-insoluble phase ("oil phase") dispersed therein. The "oil phase" can contain oil, silicone or mixtures thereof, and includes but is not limited to the oils and silicones described above in the section on water-in-oil emulsions. The distinction of whether the emulsion is characterized as an oil-in-water or silicone-in-water emulsions is a function of whether the oil phase is composed of primarily oil or silicone. The water phase of these emulsions consists primarily of water, but can also contain various other ingredients such as those water phase ingredients listed in the above section on water-in-oil emulsion. The preferred oil-in-water emulsions comprises from about 25% to about 98%, preferably from about 65% to about 95%, more preferably from about 70% to about 90% water by weight of the total composition.

In addition to a continuous water phase and dispersed oil or silicone phase, these oil-in-water compositions also comprise an emulsifier to stabilize the emulsion. Emulsifiers useful herein are well known in the art, and include nonionic, anionic, cationic, and amphoteric emulsifiers. Non-limiting examples of emulsifiers useful in the oil-in-water emulsions of this invention are given in McCutcheon's, Detergents and Emulsifiers, North American Edition (1986), published by Allured Publishing Corporation; U.S. Patent 5,011,681; U.S. Patent 4,421,769; and U.S. Patent 3,755,560.

### 4. Additional Optional Ingredients

The compositions applied in the present invention may contain a variety of other ingredients that are conventionally used in given product types provided that they do not unacceptably alter the benefits of the invention. These ingredients should be included in a safe and effective amount for a personal care composition for application to skin..

The CTFA Cosmetic Ingredient Handbook, Second Edition (1992) describes a wide variety of nonlimiting cosmetic and pharmaceutical ingredients commonly used in the skin care industry, which are suitable for use in the compositions applied in the present invention. Examples of these ingredient classes include: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, fatty alcohols and fatty acids, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents, skin-conditioning agents, skin soothing and/or healing agents and derivatives, skin treating agents, thickeners, and vitamins and derivatives thereof. Additional non-limiting examples of additional suitable skin treatment actives are included in U.S. 2003/0082219 in Section I (i.e. hexamidine, zinc oxide, and niacinamide); U.S. 5,665,339 at Section D (i.e. coolants, skin conditioning agents, sunscreens and pigments, and medicaments); and US 2005/0019356 (i.e. desquamation actives, anti-acne actives, chelators, flavonoids, and antimicrobial and antifungal actives). Examples of suitable emulsifiers and surfactants can be found in, for example, U.S. Patent 3,755,560, U.S. Patent 4,421,769, and McCutcheon's Detergents and Emulsifiers, North American Edition, pages 317-324 (1986). It should be noted, however, that many materials may provide more than one benefit, or operate via more than one mode of action. Therefore, classifications herein are made for the sake of convenience and are not intended to limit the active to that particular application or applications listed. Useful optional ingredients include:

### a. Anti-Wrinkle Actives and/or Anti-Atrophy Actives

In another embodiment the composition comprises one or more anti-wrinkle actives or anti-atrophy actives. Exemplary anti-wrinkle/anti-atrophy actives suitable for use in the compositions of the present invention include hydroxy acids (e.g., salicylic acid, glycolic acid), keto acids (e.g., pyruvic acid), ascorbic acid (vitamin C), phytic acid, lysophosphatidic acid, flavonoids (e.g., isoflavones, flavones, etc.), stilbenes, cinnamates, resveratrol, kinetin, zeatin, dimethylaminoethanol, peptides from natural sources (e.g., soy peptides), salts of sugar acids (e.g., Mn gluconate), and retinoids which enhance the keratinous tissue appearance benefits of the present invention, especially in regulating keratinous tissue condition, e.g., skin condition, and other vitamin B compounds (e.g., thiamine (vitamin B1), pantothenic acid (vitamin B5), carnitine (vitamin Bt), riboflavin (vitamin B2), and their derivatives and salts (e.g., HCl salts or calcium salts)).

### b. Anti-Oxidants and/or Racial Scavengers

In another embodiment the composition comprises an anti-oxidant/radical scavenger. The anti-oxidant/radical scavenger is especially useful for providing protection against UV radiation that can cause increased scaling or texture changes in the stratum corneum and against other environmental agents, which can cause skin damage. The anti-oxidant/radical scavenger may be from about 0.01% to about 10%, or from about 0.1% to about 5%, of the composition.

Anti-oxidants/radical scavengers such as ascorbic acid (vitamin C) and its salts, ascorbyl esters of fatty acids, ascorbic acid derivatives (e.g., magnesium ascorbyl phosphate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the tradename Trolox^{R}), amines (e.g., N,N-diethylhydroxylamine, amino-guanidine), nordihydroguaiaretic acid, bioflavonoids, amino acidssilymarin, tea extracts, and grape skin/seed extracts may be used. Preferred anti-oxidants/radical scavengers are selected from esters of tocopherol, more preferably tocopherol acetate.

### c. Additional Anti-Inflammatory Agents

In another embodiment the composition comprises anti-inflammatory at from about 0.01% to about 10%, more preferably from about 0.5% to about 5%, of the composition. The anti-inflammatory agent enhances the skin appearance benefits of the present invention, e.g., such agents contribute to a more uniform and acceptable skin tone or color. The exact amount of anti-inflammatory agent to be used in the compositions will depend on the particular anti-inflammatory agent utilized since such agents vary widely in potency.

Steroidal anti-inflammatory agents, include but are not limited to, corticosteroids such as hydrocortisone. A second class of anti-inflammatory agents, which is useful in the compositions, includes the nonsteroidal anti-inflammatory agents. The varieties of compounds encompassed by this group are well known to those skilled in the art. Specific non-steroidal anti-inflammatory agents useful in the composition invention include, but are not limited to, salicylates, flufenamic acid, etofenamate, aspirin, and mixtures thereof.

Additional anti-inflammatory agents useful herein include allantoin and compounds of the Licorice (the plant genus/species Glycyrrhiza glabra) family, including glycyrrhetic acid, glycyrrhizic acid, and derivatives thereof (e.g., esters).

### d. Anti-Cellulite Agents

In another embodiment the composition comprises an anti-cellulite agent. Suitable agents may include, but are not limited to, xanthine compounds (e.g., caffeine, theophylline, theobromine, and aminophylline).

### e. Tanning Actives

In another embodiment the composition comprises a tanning active. When present, it is preferable that the compositions comprise from about 0.1% to about 20%, more preferably from about 2% to about 7%, and even more preferably from about 3% to about 6%, by weight of the composition, of a tanning active. A preferred tanning active is dihydroxyacetone.

### f. Skin Lightening Agents

The compositions applied in the present invention may comprise a skin lightening agent from about 0.1% to about 10%, more preferably from about 0.2% to about 5%, also preferably from about 0.5% to about 2%, by weight of the composition, of a skin lightening agent. Suitable skin lightening agents include those known in the art, including kojic acid, arbutin, tranexamic acid, ascorbic acid and derivatives thereof (e.g., magnesium ascorbyl phosphate or sodium ascorbyl phosphate, ascorbyl glucoside, and the like). Other skin lightening materials suitable for use herein include Acitwhite ® (Cognis), Emblica ® (Rona), Azeloglicina (Sinerga) and extracts (e.g. mulberry extract).

### g. Sunscreen Actives

The compositions applied in the subject invention may optionally contain a sunscreen active at from about 1% to about 20%, more typically from about 2% to about 10% by weight of the composition. As used herein, "sunscreen active" includes both sunscreen agents and physical sunblocks. Suitable sunscreen actives may be organic or inorganic.

A wide variety of conventional sunscreen actives are suitable for use herein. Sagarin, et al., at Chapter VIII, pages 189 et seq., of Cosmetics Science and Technology (1972), discloses numerous suitable actives. Particularly suitable sunscreen agents are 2-ethylhexyl-p-methoxycinnamate (commercially available as PARSOL MCX), 4,4'-t-butyl methoxydibenzoylmethane (commercially available as PARSOL 1789), 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxy-propyl))aminobenzoate, 2-ethylhexyl-2-cyano-3,3-diphenylacrylate, 2-ethylhexyl-salicylate, glyceryl-p-aminobenzoate, 3,3,5-tri-methylcyclohexylsalicylate, methylanthranilate, p-dimethyl-aminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, octocrylene, zinc oxide, titanium dioxide, and mixtures thereof.

### h. Conditioning Agents

The compositions applied in the present invention may comprise a conditioning agent selected from the group consisting of humectants, moisturizers, or skin conditioners, each can be present at a level of from about 0.01% to about 40%, more preferably from about 0.1% to about 30%, and even more preferably from about 0.5% to about 15% by weight of the composition. These materials include, but are not limited to, guanidine; urea; glycolic acid and glycolate salts (e.g. ammonium and quaternary alkyl ammonium); lactic acid and lactate salts (e.g., ammonium and quaternary alkyl ammonium); aloe vera in any of its variety of forms (e.g., aloe vera gel); polyhydroxy compounds such as sorbitol, mannitol, glycerol, hexanetriol, butanetriol, propylene glycol, butylene glycol, hexylene glycol and the like; polyethylene glycols; sugars (e.g., melibiose) and starches; sugar and starch derivatives (e.g., alkoxylated glucose, fructose, sucrose, etc.); hyaluronic acid; lactamide monoethanolamine; acetamide monoethanolamine; sucrose polyester; petrolatum; and mixtures thereof.

Suitable moisturizers, also referred to in the present invention as humectants, include urea, guanidine, glycolic acid and glycolate salts (e.g. ammonium and quaternary alkyl ammonium), lactic acid and lactate salts (e.g. ammonium and quaternary alkyl ammonium), aloe vera in any of its variety of forms (e.g. aloe vera gel), polyhydroxy alcohols (such as sorbitol, glycerol, hexanetriol, propylene glycol, hexylene glycol and the like), polyethylene glycol, sugars and starches, sugar and starch derivatives (e.g. alkoxylated glucose), hyaluronic acid, lactamide monoethanolamine, acetamide monoethanolamine, and mixtures thereof.

### i. Thickening Agents (including thickeners and gelling agents)

The compositions applied in the present invention can comprise one or more thickening agents, preferably from about 0.05% to about 10%, more preferably from about 0.1% to about 5%, and even more preferably from about 0.25% to about 4%, by weight of the composition. Nonlimiting classes of thickening agents include those selected from the group consisting of: Carboxylic Acid Polymers (crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol); Crosslinked Polyacrylate Polymers (including both cationic and nonionic polymers, such as described in U. S. Patent No. 5,100,660; 4,849,484; 4,835,206; 4,628,078; 4,599,379, and EP 228,868); Polymeric sulfonic acid (such as copolymers of acryloyldimethyltaurate and vinylpyrrolidone) and hydrophobically modified polymeric sulfonic acid (such as crosspolymers of acryloyldimethyltaurate and beheneth-25 methacrylate); Polyacrylamide Polymers (such as nonionic polyacrylamide polymers including substituted branched or unbranched polymers such as polyacrylamide and isoparaffin and laureth-7 and multi-block copolymers of acrylamides and substituted acrylamides with acrylic acids and substituted acrylic acids); Polysaccharides (nonlimiting examples of polysaccharide gelling agents include those selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof); Gums (i.e. gum agents such as acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof); and crystalline, hydroxyl-containing fatty acids, fatty esters or fatty waxes (such as microfibrous bacterial cellulose structurants as disclosed in U.S. Patent Nos. 6,967,027 to Heux et al.*;* 5,207,826 to Westland et al.*;* 4,487,634 to Turbak et al.*;* 4,373,702 to Turbak et al. and 4,863,565 to Johnson et al.*,* U.S. Pat. Publ. No. 2007/0027108 to Yang et al.)

### j. Water-Soluble Vitamins

The compositions applied in the present invention may contain a safe and effective amount of one or more water soluble vitamins. Examples of water soluble vitamins include, but are not limited to, water-soluble versions of vitamin B, vitamin B derivatives, vitamin C, vitamin C derivatives, vitamin K, vitamin K derivatives, vitamin D, vitamin D derivatives, vitamin E, vitamin E derivatives, and mixtures thereof. The vitamin compounds may be included as the substantially pure material, or as an extract obtained by suitable physical and/or chemical isolation from natural (e.g., plant) sources. When vitamin compounds are present in the compositions applied in the instant invention, the compositions preferably contain from about 0.0001% to about 50%, more preferably from about 0.001 % to about 10%, still more preferably from about 0.01 % to about 5%, and still more preferably from about 0.1% to about 5%, by weight of the composition, of the vitamin compound.

### k. Particulate Material

The compositions applied in the present invention may contain one or more particulate materials. Nonlimiting examples of particulate materials useful in the present invention include colored and uncolored pigments, interference pigments, inorganic powders, organic powders, composite powders, optical brightener particles, and combinations thereof. These particulates can be platelet shaped, spherical, elongated or needle-shaped, or irregularly shaped, surface coated or uncoated, porous or non-porous, charged or uncharged, and can be added to the current compositions as a powder or as a pre-dispersion. These particulate materials may provide a wide range of functions, including but not limited to modifying skin feel, masking the appearance of certain skin characteristics such as exfoliating benefits, blotchy areas, age spots, freckles, fine lines, wrinkles, and pores, absorbing excess skin sebum/oils, reducing skin shine, improving application properties of the composition, masking the color of other components of the composition, filling in skin pores, lines and wrinkles, and reducing migration of liquid materials on the skin. Preferably, particulate materials are present in the composition in levels of from about 0.01% to about 20%, more preferably from about 0.05% to about 10%, still more preferably from about 0.1% to about 5%, by weight of the composition. There are no specific limitations as to the pigment, colorant or filler powders used in the composition. Examples of suitable particulates for use herein are described in U.S. Patent Publ. 2005/0019356A1.

### 5. Composition Forms

The topical compositions applied in the subject invention, including but not limited to lotions, milks, mousses, serums, sprays, aerosols, foams, sticks, pencils, gels, creams and ointments, may comprise a dermatologically acceptable emollient. Such compositions preferably contain from about 2% to about 50% of the emollient. As used herein, "emollient" refers to a material useful for the prevention or relief of dryness, as well as for the protection of the skin. A wide variety of suitable emollients is known and may be used herein. Sagarin, Cosmetics, Science and Technology, 2nd Ed, v1, pp. 32-43 (1972), contains numerous examples of materials suitable as an emollient. Non-limiting examples of preferred emollients include glycerin and fatty acid esters. The emollient can be used in an amount of from about 0.001 to about 20%, or from about 0.01 to about 15%, or from about 0.1 to about 10% by weight of the composition.

The physical form of the cleansing compositions is not critical. The compositions can be, for example, formulated as toilet bars, liquids, shampoos, bath gels, hair conditioners, hair tonics, pastes, or mousses. Toilet bars are preferred since this is the form of cleansing agent most commonly used to wash the skin. Rinse-off cleansing compositions, such as shampoos, require a delivery system adequate to deposit sufficient levels of actives on the skin and scalp. A preferred delivery system involves the use of insoluble complexes. See U.S. Patent 4,835,148.

The compositions applied in the present invention may also be in the form of cosmetics. Suitable cosmetic forms include, but are not limited to, foundations, lipsticks, rouges, mascaras, and the like. Such cosmetic products may include conventional ingredients such as oils, colorants, pigments, emollients, fragrances, waxes, stabilizers, and the like. Exemplary carriers and such other ingredients suitable for use herein are described, for example, in U.S. Pat. 6,060,547.

### 6. Optional Lathering Surfactants

Where the personal care composition is a wash or cleansing composition, the carrier can comprise one or more lathering surfactants and the carrier can be at a level of from about 60% to about 99.99%. A lathering surfactant defined herein as surfactant, which when combined with water and mechanically agitated generates a foam or lather. Preferably, these surfactants or combinations of surfactants should be mild, which means that these surfactants provide sufficient cleansing or detersive benefits but do not overly dry the skin or hair while still lathering. Those of skill in the art should understand that the lathering surfactant is in addition to the surfactant derived from a predominantly unsaturated triglyceride described above.

A wide variety of lathering surfactants are useful herein and include those selected from the group consisting of anionic lathering surfactants, nonionic lather surfactants, amphoteric lathering surfactants, and mixtures thereof. Generally, the lathering surfactants are fairly water soluble. When used in the composition, at least about 4% of the lathering surfactants have a HLB value greater than about ten. Examples of such surfactants are found in and U.S. Pat. 5,624,666. Cationic surfactants can also be used as optional components, provided they do not negatively impact the overall lathering characteristics of the required lathering surfactants

Concentrations of these surfactant are from about 10% to about 20%, alternatively from about 6% to about 25%, and alternatively from about 4% to about 30% by weight of the composition. To avoid skin irritation issues, the compositions should have a ratio by weight of the composition of anionic surfactant to amphoteric and/or zwitterionic surfactant is from about 1.1: 1 to about 1:1.5, alternatively from about 1.25:1 to about 1:2, and alternatively from about 1.5:1 to about 1:3.

Anionic lathering surfactants useful in the compositions of the present invention are disclosed in McCutcheon's, Detergents and Emulsifiers, North American edition (1986), published by allured Publishing Corporation; McCutcheon's, Functional Materials, North American Edition (1992); and U.S. Pat. No. 3,929,678. A wide variety of anionic lathering surfactants are useful herein. Non-limiting examples of anionic lathering surfactants include those selected from the group consisting of sarcosinates, sulfates, sulfonates, isethionates, taurates, phosphates, lactylates, glutamates, and mixtures thereof.

Other anionic materials useful herein are soaps (i.e., alkali metal salts, e.g., sodium or potassium salts) of fatty acids, typically having from about 8 to about 24 carbon atoms, preferably from about 10 to about 20 carbon atoms, monoalkyl, dialkyl, and trialkylphosphate salts, alkanoyl sarcosinates corresponding to the formula RCON(CH₃)CH₂CH₂CO₂M wherein R is alkyl or alkenyl of about 10 to about 20 carbon atoms, and M is a water-soluble cation such as ammonium, sodium, potassium and alkanolamine (e.g., triethanolamine). Also useful are taurates which are based on taurine, which is also known as 2-aminoethanesulfonic acid, and glutamates, especially those having carbon chains between C₈ and C₁₆.

Non-limiting examples of preferred anionic lathering surfactants useful herein include those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof.

Suitable amphoteric or zwitterionic detersive surfactants for use in the compositions herein include those which are known for use in hair care or other personal care cleansing. Concentration of such amphoteric detersive surfactants is from about 1% to about 10%, alternatively from about 0.5 % to about 20% by weight of the composition. Non-limiting examples of suitable zwitterionic or amphoteric surfactants are described in U.S. Patents 5,104,646 and U.S. Patent 5,106,609.

Nonionic lathering surfactants for use in the compositions applied in the present invention are disclosed in McCutcheon's, Detergents and Emulsifiers, North American edition (1986), published by allured Publishing Corporation; and McCutcheon's, Functional Materials, North American Edition (1992); both of which are incorporated by reference herein in their entirety. Nonionic lathering surfactants useful herein include those selected from the group consisting of alkyl glucosides, alkyl polyglucosides, polyhydroxy fatty acid amides, alkoxylated fatty acid esters, lathering sucrose esters, amine oxides, and mixtures thereof.

Other examples of nonionic surfactants include amine oxides. Amine oxides correspond to the general formula R¹R²R³NO, wherein R¹ contains an alkyl, alkenyl or monohydroxy alkyl radical of from about 8 to about 18 carbon atoms, from 0 to about 10 ethylene oxide moieties, and from 0 to about 1 glyceryl moiety, and R² and R³ contain from about 1 to about 3 carbon atoms and from 0 to about 1 hydroxy group, e.g., methyl, ethyl, propyl, hydroxyethyl, or hydroxypropyl radicals. Examples of amine oxides suitable for use in this invention include dimethyl-dodecylamine oxide, oleyldi(2-hydroxyethyl) amine oxide, dimethyloctylamine oxide, dimethyl-decylamine oxide, dimethyl-tetradecylamine oxide, 3,6,9-trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

Preferred lathering surfactants for use herein are the following, wherein the anionic lathering surfactant is selected from the group consisting of ammonium lauroyl sarcosinate, sodium trideceth sulfate, sodium lauroyl sarcosinate, sodium myristoyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cetyl sulfate, sodium lauroyl lactylate, triethanolamine lauroyl lactylate, and mixtures thereof; wherein the nonionic lathering surfactant is selected from the group consisting of lauramine oxide, cocoamine oxide, decyl polyglucose, lauryl polyglucose, sucrose cocoate, C₁₂₋₁₄ glucosamides, sucrose laurate, and mixtures thereof; and wherein the amphoteric lathering surfactant is selected from the group consisting of disodium lauroamphodiacetate, sodium lauroamphoacetate, cetyl dimethyl betaine, cocoamidopropyl betaine, cocoamidopropyl hydroxy sultaine, and mixtures thereof.

### 7. Methods of Use

The personal care composition can be in any suitable personal care composition which comes in contact with skin or hair. Non-limiting examples of suitable personal care compositions include cosmetics, moisturizers, lotions, oils, personal cleansers, facial cleansers, shave gels, shave foams, shave oils, after shaves, pre-shave treatments such as lotions, and so forth. The present composition can be used in combination with various hair removal applications (prior to, concurrently with, and/or after), including but not limited to shaving (wet or dry shaving, via electric razors, via powered or manual razors which can be reusable or disposable, and combinations thereof), epilation, electrolysis, wax or depilatories as well as energy delivery devices to help regulate hair growth. Nonlimiting examples of energy deliver devices include: light, heat, sound (including ultrasonic waves and radio frequency), electrical energy, magnetic energy, electromagnetic energy (including radiofrequency waves and microwaves), and combinations thereof. The light energy may be delivered by devices including, but not limited to, lasers, diode lasers, diode laser bars, diode laser arrays, flash lamps, intense pulsed light (IPL) sources, and combinations thereof. *See e.g.* US2006/0235370A1.

In one preferred embodiment, the personal care composition is used as a post shave moisturizers and/or balms. The present invention also relates to a method of reducing irritation by down regulating cytokine activity by applying a personal care composition of the present invention onto skin to form a treated surface. Where the composition is used in a shaving regimen, the method of use can further comprise a step of shaving a portion of skin which can be performed before or after applying the composition to skin. In one preferred embodiment, the personal care composition is used in a post-shave application such as a leave-on gels, balm, or moisturizer to be applied to skin immediately after or shortly after shaving. Those of skill in the art will understand that the hair removal step can be shaving or any of the hair removal technologies described in the previous paragraph. The composition can be left on for a brief or extended amount of time (i.e. not washed off), for example from 1 minute to 24 hours, or from about 5 minutes to about 3 hour, or from about 10 minutes to about 20 minutes.

### 8. In Vitro Assay Testing

The following *in vitro* assay test was conducted to determine the effect of White Tea Extract, Panthenol and Glycyrrhizinate salt on cytokine expression in hTERT keratinocytes:
BACKGROUND: hTERT-keratinocytes are a telomerase-immortalized neonatal Caucasian epidermal keratinocyte line. Cell cultures of this type are known in the literature and have been used as models for multiple pathways as shown in the following references: Shay and Wright; Carcinogenesis, vol 26; no 5, pp 867-74, 2005; Bodner, et. al., Science, vol 279 pp 349-52, 1998; Ramirez, et. al., Oncogene, 22; pp 433-44, 2002. These cells display many features characteristic of normal primary keratinocytes and respond to normal growth controls *in vitro.*
TEST PROTOCOL: all measures were run in replicate (n=6).

To ensure proper levels of the biological active molecules for the *in vitro* study, dilution studies are conducted and ATP is analyzed for the highest non-cytotoxic concentration. White Tea Extract (H1615 WS, lot 98680094S-005) at a level of 500 ppm, Panthenol (lot 10063909S-018) at a level of 500 ppm, and Glycyrrhizinate salt (Barnet 25908N297) at a level of 500 ppm are used for this study. ATP analysis is described below.

ATP analysis is conducted using Cell Titer-Glo. The CellTiter-Glo Luminescent Cell Viability Assay is obtained from Promega Corporation (Madison, WI). This assay is a homogeneous method for determining the number of viable cells in culture based on quantitation of ATP present, signaling the presence of metabolically active cells. After the final treatment and supernatants collected, the CellTiter-Glo reagent is added. The reagent is then transferred to a BD Falcon (Franklin Lakes, NJ) Microtest 96-well Assay Plate (black, flat bottom, reference 353945, lot 041151). Luminescence is read on a Perkin Elmer Fluorimeter (Wallac Envision 2101 Multilabel Reader). Relative luminescence units are plotted in percent control versus treatment concentration.

hTERT cells are cultured in complete EpiLife (Cascade Biologics, Portland, OR) culture media in T75 culture flasks (Falcon, Becton Dickinson Labware, Franklin Lakes, NJ) under 37°C / 5% CO₂ conditions. When the flasks reach 60-80% confluence, the cells are removed from the flasks with mild enzymatic treatment according to the manufacturer's suggestions (Cascade Biologics), washed and resuspended in EpiLife Culture Medium at a concentration of 10⁵/ml. 2 ml of the cell suspension (density 20,000/cm²) are plated in each well of a 6-well culture dish (Falcon) and cultured under 37°C / 5% CO₂ conditions overnight. A complete media exchange is performed and the cells are again cultured overnight under 37°C / 5% CO₂ conditions. The culture media is removed and 2 ml of treatment are placed in each well and the cells are cultured for 18 hrs. For all legs other than Control, IL-1β is added to the treatment at 1 nanogram per mL (for 2 nanograms per treatment). After the treatment period, the cell culture supernatants are collected and analyzed immediately for Interleukin-1α with an R&D Systems (Minneapolis, MN) kit which deploys the quantitative sandwich immunoassay technique. ATP is measured from cell lysates an indicator of viability. IL-1α is indexed to cell viability to ensure a proper reading of actual activity.

TESTING: This test was conducted and the analysis of the data is as follows. Statistical differences among treatments for IL-1α were analyzed via JMP using a log-transform before the analysis of variance. The log-transform ensures that the response data are normally distributed and have equal variance.

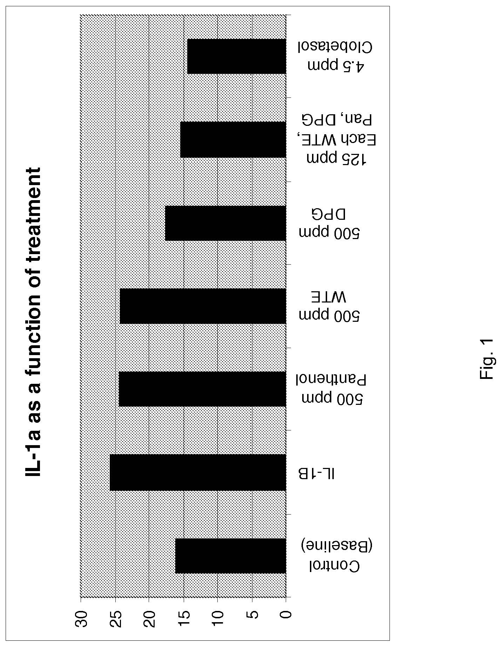

Surprisingly, as shown in the FIG. 1 and Table A, below, the combination of extract of *camellia sinesis* (in this case white tea extract), panthenol and glycyrrhizinate salt at 125 ppm each (375 ppm total) exhibit significant synergistic activity reducing IL-1α (irritation marker) relative to the materials alone at 500 ppm each. This is illustrated in Table A in the significance groupings where 500 ppm of white tea extract (WTE) and 500 ppm panthenol (Pan) are not significantly different than the IL-1β treated cells (noted by the letter A). The 500 ppm glycyrrhizinate salt (DPG) provides slightly higher IL-1α reduction in the IL-1β treated cells compared to 500 ppm of white tea extract and 500 ppm panthenol. Furthermore, the combination of these actives at 125 ppm each (for a total of 375 ppm of the multi-active system) is measurably better than 500 ppm of any one of these actives alone, showing that the present invention achieves better IL-1α reduction even at a lower level. Even more surprising, the IL1-α reduction achieved with the multi-active system was close to the reduction achieved by a sample treated with Clobetasol (a high benchmark prescription anti-inflammatory used as a positive control in this test) for reducing IL-1α. Note, 125 ppm represents a 2x dilution of the 500 ppm material - 500 to 250 to 125.

In Table A, the "Control" leg represents the average IL-1α level in the cells themselves, "IL-1β" addition increases the IL-1α level then individual and the combination of ingredients were dosed to IL-1β treated cells. The average score is represented by 4 replicates for the control leg and 6 replicates from the IL-1β and treatment legs. Statistical Grouping levels not connected by same letter are significantly different at > 95% confidence.

**Table A**

| **Leg** | **Level and Ingredient** | **Statistical Grouping** | | | | **Avg IL-1α measurement** |
|---|---|---|---|---|---|---|
| 1 | Control (Baseline) | | B | C | | 16.12 |
| 2 | IL-1β | A | | | | 25.65 |
| 3 | 500 ppm Panthenol + IL-1β | A | | | | 24.41 |
| 4 | 500 ppm White Tea Extract + IL-1β | A | | | | 24.26 |
| 5 | 500 ppm Glycyrrhizinate salt + IL-1β | | B | | | 17.57 |
| 6 | 125 ppm of the Multi-active System (White Tea, Panthenol, Glycyrrhizinate salt) + IL-1β | | | C | D | 15.33 |
| 7 | 4.5 ppm Clobetasol + IL-1β | | | | D | 14.29 |

Without intending to be bound by theory, Applicants have found that when the present multi-active system is used in combination, a resultant decrease in IL-1α cytokine levels is demonstrated. Applicants believe that the reduction in IL-1α levels from the combination can lead to a reduction in shave-induced irritation.

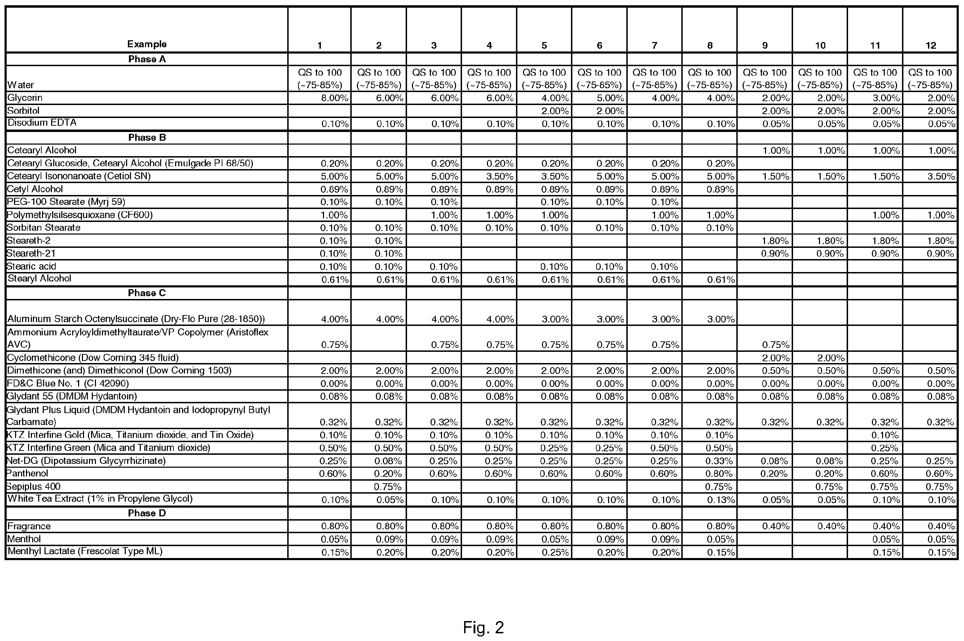

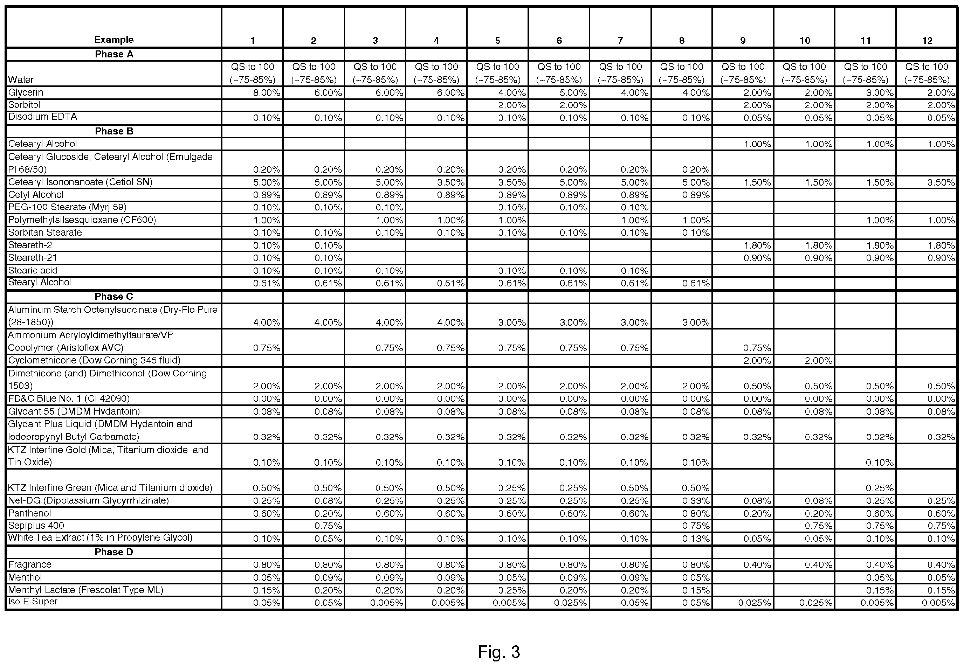

### 9. Examples and FIGs. 2 and 3

Examples shown in FIG. 2 and FIG 3 are: Moisturizer/Balms. These examples are non-limiting and other product forms, such as those described above are also within the scope of the invention. These examples are made as follows:
Phase A materials are combined and heated in a container. Phase B materials are combined and heated in a separate container. Phase B is added to Phase A under high shear. The mixture of Phases A and B is cooled and the contents of Phase C are added with mixing. Phase D materials are blended in a separate container and added to the mixture of Phases A, B, and C. The final mixture is stirred until well blended. Qs means quantity sufficient to reach 100%. All examples in FIGs. 2 and 3 are in accordance with the present invention.

All percentages and ratios used herein are by weight of the total composition and all measurements made are at 25°C, unless otherwise designated.

The compositions applied in the present invention can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

"Dermatologically acceptable," as used herein, means that the compositions or components described are suitable for use in contact with human keratinous tissue without undue toxicity, incompatibility, instability, allergic response, and the like.

All percentages disclosed herein, unless otherwise stated, are by weight of the named material itself that is found in the compositions, thereby excluding for example the weight associated with carriers, impurities and by-products found in the raw material.

It should be understood that every maximum numerical limitation given throughout this specification includes every lower numerical limitation, as if such lower numerical limitations were expressly written herein. Every minimum numerical limitation given throughout this specification includes every higher numerical limitation, as if such higher numerical limitations were expressly written herein. Every numerical range given throughout this specification includes every narrower numerical range that falls within such broader numerical range, as if such narrower numerical ranges were all expressly written herein.

All parts, ratios, and percentages herein, in the Specification, Examples, and Claims, are by weight and all numerical limits are used with the normal degree of accuracy afforded by the art, unless otherwise specified.

Except as otherwise noted, the articles "a," "an," and "the" mean "one or more."

## Claims

1. A method of applying a personal care composition to a portion of the skin to form a treated surface, said composition comprising:
a) from 0.001% to 8%, preferably from 0.1% to 3% by weight of a multi-active system comprising:
i) at least 5% to 50% by weight of said multi-active system of an extract of *camellia sinensis;*
ii) at least 15% to 80% by weight of said multi-active system of panthenol; and
iii) at least 5% to 50% by weight of said multi active system of glycyrrhizinate salt; and
b) from 50% to 99.99% of a carrier, and further comprising a step of at least partially removing hair from said at least a portion of the treated surface.

2. The method of any preceding claim, wherein the extract of *camellia sinensis* comprises a white tea extract.

3. The method of any preceding claim, wherein the glycyrrhizinate salt is a dipotassium glycyrrhizinate salt.

4. The method of any preceding claim, wherein said multi-active system comprises a ratio of said extract of *camellia sinensis* to glycyrrhizinate salt to panthenol is from 10:5:1 to 1:5:10 by weight.

5. The method of any preceding claim, wherein the carrier is selected from the group consisting of an oil-in-water emulsion and a water-in-oil emulsion, wherein the oil phase preferably comprises silicone.

6. The method of any preceding claim, wherein the carier further comprises from 4% to 30% by weight of a lathering surfactant by weight of the composition.

7. The method of any preceding claim, further comprising a step of at least partially removing hair from a portion of skin, prior to the step of applying said personal care composition to said portion of skin.

8. The method of any preceding claim, wherein the personal care composition is not washed off after application on the treated surface, and is left on the treated surface for at least one minute to 24 hours.

## Patentansprüche

1. Verfahren zum Auftragen einer Körperpflegezusammensetzung auf einen Hautabschnitt, um eine behandelte Oberfläche zu bilden, wobei die Zusammensetzung Folgendes umfasst:
a) von 0,001 Gew.% bis 8 Gew.-%, vorzugsweise von 0,1 Gew.-% bis 3 Gew.-% eines mehrfach aktiven Systems, umfassend:
i) zu mindestens 5 Gew.-% bis 50 Gew.-% des mehrfach aktiven Systems einen Extrakt von *Camellia sinensis;*
ii) zu mindestens 15 Gew.-% bis 80 Gew.-% des mehrfach aktiven Systems Panthenol; und
iii) zu mindestens 5 Gew.-% bis 50 Gew.-% des mehrfach aktiven Systems Glycyrrhizinatsalz; und
b) von 50 % bis 99,99 % einen Träger, und ferner umfassend einen Schritt des wenigstens teilweisen Entfernens von Haar von wenigstens einem Teil der behandelten Oberfläche.

2. Verfahren nach einem der vorstehenden Ansprüche, wobei der Extrakt von *Camellia sinensis* einen Weißtee-Extrakt umfasst.

3. Verfahren nach einem der vorstehenden Ansprüche, wobei das Glycyrrhizinatsalz ein Dikaliumglycyrrhizinatsalz ist.

4. Verfahren nach einem der vorstehenden Ansprüche, wobei das mehrfach aktive System ein Verhältnis des Extrakts von *Camellia sinensis* zu Glycyrrhizinatsalz zu Panthenol von 10:5:1 bis 1:5:10, bezogen auf das Gewicht, umfasst.

5. Verfahren nach einem der vorstehenden Ansprüche, wobei der Träger ausgewählt ist aus der Gruppe bestehend aus einer Öl-in-Wasser-Emulsion und einer Wasser-in-Öl-Emulsion, worin die Ölphase vorzugsweise Silikon umfasst.

6. Verfahren nach einem der vorstehenden Ansprüche, wobei der Träger ferner von 4 Gew.-% bis 30 Gew.-% ein schaumerzeugendes Tensid, bezogen auf das Gewicht der Zusammensetzung, umfasst.

7. Verfahren nach einem der vorstehenden Ansprüche, ferner umfassend einen Schritt des wenigstens teilweisen Entfernens von Haar von einem Hautabschnitt vor dem Schritt des Auftragens der Körperpflegezusammensetzung auf den Hautabschnitt.

8. Verfahren nach einem der vorstehenden Ansprüche, wobei die Körperpflegezusammensetzung nach dem Auftragen auf die behandelte Oberfläche nicht abgewaschen wird und für mindestens eine Minute bis 24 Stunden auf der behandelten Oberfläche belassen wird.

## Revendications

1. Procédé d'application d'une composition de soins personnels sur une partie de la peau pour former une surface traitée, ladite composition comprenant :
a) de 0,001 % à 8 %, de préférence de 0,1 % à 3 % en poids d'un système à plusieurs agents actifs, comprenant :
i) au moins 5 % à 50 % en poids dudit système à plusieurs agents actifs d'un extrait de *camellia sinensis* ;
ii) au moins 15 % à 80 % en poids dudit système à plusieurs agents actifs de panthénol ; et
iii) au moins 5 % à 50 % en poids dudit système à plusieurs agents actifs de sel de glycyrrhizinate ; et
b) de 50 % à 99,99 % d'un véhicule, et comprenant en outre une étape consistant à éliminer au moins partiellement les poils de ladite au moins une partie de la surface traitée.

2. Procédé selon l'une quelconque des revendications précédentes, dans lequel l'extrait de *camellia sinensis* comprend un extrait de thé blanc.

3. Procédé selon l'une quelconque des revendications précédentes, dans lequel le sel de glycyrrhizinate est un sel de glycyrrhizinate dipotassique.

4. Procédé selon l'une quelconque des revendications précédentes, dans lequel ledit système à plusieurs agents actifs comprend un rapport dudit extrait de *camellia sinensis* au sel de glycyrrhizinate au panthénol allant de 10:5:1 à 1:5:10 en poids.

5. Procédé selon l'une quelconque des revendications précédentes, dans lequel le véhicule est choisi dans le groupe constitué d'une émulsion huile-dans-eau et d'une émulsion eau-dans-huile, dans lequel la phase huileuse comprend de préférence de la silicone.

6. Procédé selon l'une quelconque des revendications précédentes, dans lequel le véhicule comprend en outre de 4 % à 30 % en poids d'un agent tensioactif moussant en poids de la composition.

7. Procédé selon l'une quelconque des revendications précédentes, comprenant en outre une étape consistant à éliminer au moins partiellement les poils d'une partie de peau, avant l'étape d'application de ladite composition de soins personnels sur ladite partie de peau.

8. Procédé selon l'une quelconque des revendications précédentes, dans lequel la composition de soins personnels n'est pas éliminée par lavage après application sur la surface traitée, et est laissée sur la surface traitée pendant au moins une minute à 24 heures.
